# EUROPEAN PATENT SPECIFICATION

(11) **EP 1 721 966 B1**
(45) Date of publication and mention of the grant of the patent: **28.10.2009**
(21) Application number: 05381022.2
(22) Date of filing: 11.05.2005
(51) Int. Cl.: C12N 1/20, C05F 17/00, C12R 1/11, C12R 1/125

(54) **Bacterial strain and composition used to accelerate composting and as a fertilizer**
Bakterienstamm und Zusammensetzung verwendet zum beschleunigten Kompostieren sowie als Dünger
Souche bactérienne et composition utilisée pour accélérer le compostage et comme engrais

(43) Date of publication of application: 15.11.2006
(73) Proprietor: TRICHODEX, S.A., Dos Hermanas 41700 (ES)
(72) Inventor: Pérez Barrera, Francisco c/o TRICHODEX, S.A., 41700 Dos Hermanas (ES); Exposito Cubero, Carmelo c/o TRICHODEX, S.A, 41700 Dos Hermanas (ES); Gomis Garcia, M Dolores c/o TRICHODEX, S.A., 41700 Dos Hermanas (ES); Akti, Khalid c/o TRICHODEX, S.A., 41700 Dos Hermanas (ES)
(74) Representative: Esteban Perez-Serrano, Maria Isabel

(56) References cited:
- US-A- 5 464 766
- PATENT ABSTRACTS OF JAPAN vol. 2003, no. 05, 12 May 2003 (2003-05-12) & JP 2003 009848 A (KIYOMOTO BIO CO LTD; SUZUKI MITSUO), 14 January 2003 (2003-01-14)
- GAUR A C: "RECYCLING OF ORGANIC WASTES BY IMPROVED TECHNIQUES OF COMPOSTING AND OTHER METHODS" RESOURCES AND CONSERVATION, vol. 13, no. 2-4, 1987, pages 157-174, XP009054205 & INTERNATIONAL SYMPOSIUM ON RECYCLING OF ORGANIC WASTES FOR FERTILIZER, FOOD, FEED AND FUEL, HONG KON ISSN: 0166-3097
- EUSEBIO A ET AL: "Microflora evaluation of two agro-industrial effluents treated by the JACTO jet-loop type reactor system" WATER SCIENCE AND TECHNOLOGY, vol. 51, no. 1, January 2005 (2005-01), pages 107-112, XP009054197 ISSN: 0273-1223
- PATENT ABSTRACTS OF JAPAN vol. 2000, no. 11, 3 January 2001 (2001-01-03) & JP 2000 210647 A (HEIWA BUSSAN KK; ISHIDA MICHIO; RO YOSHIO), 2 August 2000 (2000-08-02)

### SEQUENCE LISTING

<110> TRICHODEX, S.A.
<120> CEPA BACTERIANA Y COMPOSICIÓN PARA SU USO EN LA ACELERACIÓN DEL COMPOSTAJE Y COMO FERTILIZANTE
<130> 20051434
<160> 2
<170> PatentIn version 3.3
<210> 1
   <211> 1487
   <212> DNA
   <213> Bacillus megaterium
<220>
   <221> misc_feature
   <222> (311)..(311)
   <223> n is a, c, g, or t
<220>
   <221> misc_feature
   <222> (1113)..(1113)
   <223> n is a, c, g, or t
<400> 1
   gctggcggcg tgcctaatac atgcaagtcg agcgaactga ttagaagctt gcttctatga      60
   cgttagcggc ggacgggtga gtaacacgtg ggcaacctgc ctgtaagact gggataactt     120
   cgggaaaccg aagctaatac cggataggat cttctccttc atgggagatg attgaaagat     180
   ggtttcggct atcacttaca gatgggcccg cggtgcatta gctagttggt gaggtaacgg     240
   ctcaccaagg caacgatgca tagccgacct gagagggtga tcggccacac tgggactgag     300
   acacggccca nactcctacg ggaggcagca gtagggaatc ttccgcaatg gacgaaagtc     360
   tgacggagca acgccgcgtg agtgatgaag gctttcgggt cgtaaaactc tgttgttagg     420
   gaagaacaag tacaagagta actgcttgta ccttgacggt acctaaccag aaagccacgg     480
   ttaactacgt gccagcagcc gtggtaatac gtaggtggca agcgttatcc ggaattattg     540
   ggcgtaaagc gcgcgcaggc ggtttcttaa gtctgatgtg aaagcccacg gctcaaccgt     600
   ggagggtcat tggaaactgg ggaacttgag tgcagaagag aaaagcggaa ttccacgtgt     660
   agcggtgaaa tgcgtagaga tgtggaggaa caccagtggc gaaggcggct ttttggtctg      720
   taactgacgc tgaggcgcga aagcgtgggg agcaaacagg attagatacc ctggtagtcc     780
   acgccgtaaa cgatgagtgc taagtgttag agggtttccg ccctttagtg ctgcagctaa     840
   cgcattaagc actccgcctg gggagtacgg tcgcaagact gaaactcaaa ggaattgacg     900
   ggggcccgca caagcggtgg agcatgtggt ttaattcgaa gcaacgcgaa gaaccttacc     960
   aggtcttgac atcctctgac aactctagag atagagcgtt ccccttcggg ggacagagtg    1020
   acaggtggtg catggttgtc gtcagctcgt gtcgtgagat gttgggttaa gtcccgcaac    1080
   gagcgcaacc cttgatctta gttgccagca ttnagttggg cactctaagg tgactgccgg    1140
   tgacaaaccg gaggaaggtg gggatgacgt caaatcatca tgccccttat gacctgggct    1200
   acacacgtgc tacaatggat ggtacaaagg gctgcaagac cgcgaggtca agccaatccc    1260
   ataaaaccat tctcagttcg gattgtaggc tgcaactcgc ctacatgaag ctggaatcgc    1320
   tagtaatcgc ggatcagcat gccgcggtga atacgttccc gggccttgta cacaccgccc    1380
   gtcacaccac gagagtttgt aacacccgaa gtcggtggag taaccgtaag gagctagccg    1440
   cctaaggtgg gacagatgat tggggtgaag tcgtaacaag gtagccg                  1487
<210> 2
   <211> 1479
   <212> DNA
   <213> Bacillus subtilis
<220>
   <221> misc_feature
   <222> (144)..(144)
   <223> n is a, c, g, or t
<220>
   <221> misc_feature
   <222> (213)..(213)
   <223> n is a, c, g, or t
<220>
   <221> misc_feature
   <222> (244)..(244)
   <223> n is a, c, g, or t
<220>
   <221> misc_feature
   <222> (255)..(255)
   <223> n is a, c, g, or t
<220>
   <221> misc_feature
   <222> (303)..(303)
   <223> n is a, c, g, or t
<400> 2
   ggcgtgccta atacatgcaa gtcgagcgga cagatgggag cttgctccct gatgttagcg     60
   gcggacgggt gagtaacacg tgggtaacct gcctgtaaga ctgggataac tccgggaaac    120
   cggggctaat accggatggt tgtntgaacc gcatggttca aacataaaag gtggcttcgg    180
   ctaccactta cagatggacc cgcggcgcat tanctagttg gtgaggtaac ggctcaccaa    240
   ggcnacgatg cgtanccgac ctgagagggt gatcggccac actgggactg agacacggcc    300
   canactccta cgggaggcag cagtagggaa tcttccgcaa tggacgaaag tctgacggag    360
   caacgccgcg tgagtgatga aggttttcgg atcgtaaagc tctgttgtta gggaagaaca    420
   agtgccgttc aaatagggcg gcaccttgac ggtacctaac cagaaagcca cggttaacta    480
   cgtgccagca gccgcggtaa tacgtaggtg gcaagcgttg tccggaatta ttgggcgtaa    540
   agggctcgca ggcggtttct taagtctgat gtgaaagccc ccggctcaac cggggagggt    600
   cattggaaac tggggaactt gagtgcagaa gaggagagtg gaattccacg tgtagcggtg    660
   aaatgcgtag agatgtggag gaacaccagt ggcgaaggcg actctctggt ctgtaactga    720
   cgctgaggag cgaaagcgtg gggagcgaac aggattagat accctggtag tccacgccgt    780
   aaacgatgag tgctaagtgt tagggggttt ccgcccctta gtgctgcagc taacgcatta    840
   agcactccgc ctggggagta cggtcgcaag actgaaactc aaaggaattg acgggggccc    900
   gcacaagcgg tggagcatgt ggtttaattc gaagcaacgc gaagaacctt accaggtctt    960
   gacatcctct gacaatccta gaagatagga cgtccccttc gggggcagag tgacaggtgg   1020
   tgcatggttg tcgtcagctc gtgtcgtgag atgttgggtt aagtcccgca acgagcgcaa   1080
   cccttgatct tagttgccag cattcagttg ggcactctaa ggtgactgcc ggtgacaaac   1140
   cggaggaagg tggggatgac gtcaaatcat catgcccctt atgacctggg ctacacacgt   1200
   gctacaatgg acagaacaaa gggcagcgaa accgcgaggt taagccaatc ccacaaatct   1260
   gttctcagtt cggatcgcag tctgcaactc gactgcgtga agctggaatc gctagtaatc   1320
   gcggatcagc atgccgcggt gaatacgttc ccgggccttg tacacaccgc ccgtcacacc   1380
   acgagagttt gtaacacccg aagtcggtga ggtaaccttt taggagccag ccgccgaagg   1440
   tgggacagat gattggggtg aagtcgtaac aaggtagcc                          1479

## Description

### OBJECT OF THE INVENTION

### DESCRIPTION

The present invention relates to the development of a composition based on natural micro-organisms, able to accelerate the composting process of agricultural organic residues, solid urban residues and sludge from residual water purification stations among others, as well as being used as a fertilizer.

The present invention relates to the enrichment of the residues with a beneficial microbial population that can intervene into, accelerate and complete the decay process in optimum time, as well as the decay of the volatile components (sulphur by-products, amines and fatty acids) responsible for the bad odour.

The composition of the present invention increases the number of micro-organisms present naturally to decay organic waste. The bacterial strains used in the composition of this invention, thanks to the creation of a battery of extracellular enzymes, are able to breakdown relatively large molecular chains (proteins, carbo-hydrates and fats) converting them into more soluble and assimilable products.

In addition, the composition can also be used as a fertilizer.

The applicant of the present invention has isolated new strains which belong to the Bacillus megaterium and Bacillus subtilis species.
The invention is characterised by a composition for accelerating composting processes which at least comprises the two strains isolated for the first time, as well as its use to accelerate fermentative processes and as a fertilizer.

### BACKGROUND OF THE INVENTION

Waste resulting from industrial and agricultural activities, as well as municipal solid waste and residual water sludge, are closely related to environmental pollution.

Currently, the management of this waste is controlled by directives and standards meant to determine the conditions in which the waste must be treated, as well as their applications and incidence on the environment, crops, living creatures and health in general. A central issue of these directives is the prohibition of using said residues without a prior treatment. An example of this is the National Plan for waste management, issued by the Secretary General for the Environment, which sets the proper management of waste and establishes the legal framework for its correct evaluation and recycling, according to European and Spanish Law. The object of the Plan is to protect the environment, particularly the quality of the ground, by means of proper waste management.

The plan follows the hierarchical principle considered in the Law on Waste of 1998, which requires recycling waste as much as possible this is, making full use of its matter before using the energy contained in it. This implies prioritising composting in all of its varieties.
The composting process involves a microbial transformation in aerobic conditions of organic matter. This process requires a solid substrate on which act various populations of micro-organisms (bacteria, actinomycetes and fungi) responsible for decomposing organic matter, to produce a stabilized organic product that can be used in agriculture to improve the soil.

Composting of waste becomes the best option for eliminating them, solving a financial and health problem by giving them a value and object.

Inoculation of waste to be composed with a microbial biomass is a strategy of vital importance to accelerate these processes.

This strategy consists of adding micro-organisms (bacteria, fungi and actinomycetes) to organic waste. These micro-organisms can be isolated from the waste or from elsewhere, and are selected for their special properties of accelerating the composting process and concluding it successfully.

The strategy begins by an exhaustive characterisation study of the micro-organisms in the inoculation in the laboratory. Vargas-Garcia et al. have studied the ability of six ligno-cellulitic fungal strains to grow in agricultural waste and produce inocula for composting. (Vargas-Garcia, M.C., López, M.J., Suárez, F. and Moreno, J. (2005), Laboratory study of inocula production for composting processes. Bioresource Technology, 96: 797-803).

In addition, Lei and VanderGheynst have studied the effect of inoculation with micro-organisms and pH on the microbial population during the composting process (Lei, F. and VanderGheynst, J.S., (2000). The effects of microbial inoculation and pH on microbial community structure changes during composting. Process Biochemistry, 35: 923-929).

Patent with publication number JP2003009848 describes a method for decaying organic matter which uses Bacillus subtilis among other micro-organisms.

In view of the above, the applicant has developed compositions based on natural micro-organisms, particularly bacteria, which in addition to enriching the substrates with a population of beneficial micro-organisms are capable of participating and accelerating the composting process in optimum periods, as well as to reduce the composting process work and allow its use as a fertilizer.

### DESCRIPTION OF THE INVENTION

The present invention relates to the isolation of a bacterial strain, a composition that accelerates the composting process and the use made of it.

The composition of the present invention includes at least two strains, isolated by the same applicant.

These strains were isolated and selected from soil samples obtained from areas around healthy plants and hosts, taken from different geographical areas; specifically, they have been isolated from onion roots.

These strains have been deposited for patent purposes in the Spanish Collection Culture Type (*Colección Española de Cultivos Tipo, CECT*) on 27 July 2004, with the following numbers assigned to them: AMCB1, CECT 7012 and AMCB3, CECT 7014.

The isolated strains were maintained with a high survival rate in a 25% glycerol solution and stored at -80 °C. The strains were easily recovered in normal laboratory culture mediums such as nutritional agar (NA), nutritional broth (NB), tryptone-soy-agar (TSA) and potato-dextrose-agar (PDA).

A number of tests were conducted to characterise the strains.

Their growth was studied in solid mediums such as TY (tryptone-yeast), nutritional agar (NA) and YM (yeast-manitol). In all cases the micro organism which grew the quickest (14h) was AMCB1.

Biochemical assays were performed five-fold to establish a reliable analysis of the bacteria regarding their growth and behaviour with different reagents.

Both stains AMCB1 and AMCB3 tested positive in the assay for the enzyme catalase.

In the assay for enzyme oxydase the strain AMCB1 tested negative and AMCB3 gave a variable result.

The capacity to reduce nitrates and nitrites was also studied.

A positive result in this test means that the micro-organism can use nitrogen in any oxidation state, but is always assimilated as the ammonium ion. Specifically, for the strains under study the result was negative form AMCB1 and positive for AMCB3. The strain AMCB3 presents nitrate reductase but not nitrite reductase enzyme.

The capacity to hydrolyse urea was also determined. The result was positive but weak for AMCB1 and negative for AMCB3.

The PGPR (Plant Growth Promoting Rhizobacteria) activity of the strains object of this patent was studied by their growth in different mediums: ACC (1-amino-cyclopropan-1-carboxylate), MLACC (medium lacking 1-amino-cyclopropan-1-carboxylate), PDYA (potato-dextrose-yeast-agar), the production of siderophora was aqlso determined and the capacity to produce auxins.

The results obtained seem to indicate that the strains AMCB1 and AMCB3 have a good PGPR activity, as they were able to grow in all media except the one with siderophora and presented a greater production of auxins. Therefore, they can exert a beneficial influence on the plant both directly, improving their nutrition by solubilising the phosphates in the medium, and indirectly by the activity of the enzyme ACC deaminase, which causes a reduction in the ethylene levels of the plant which results in an increased root length.

The fatty acid profile of the various bacterial strains was studied individually, according to the protocol described by Jarvis (1994) and Sasser (1990).

The strains AMCB1 and AMCB3 show great variability in the composition of fatty acids, as well as containing fatty acids of the type 18:2w6 or 17:1w8 characteristic of Bacillus species among others.

Studies were conducted on the assimilation of "Biolog" carbon sources.

The strain AMCB3 shows a low use of glucogen, while AMCB1 shows a high use of glucogen, with a moderate use of the remaining polymers.

As regards the use of carbohydrates, i-erytritol produced a composite in which the strains grew very little or not at all.

As with the results for glucogen, the strain AMCB1 shows the greatest growth in all mediums with glucose precursors. AMCB3 grew well only in the presence of D- galacturonic acid and pyruvic methylester.

As regards behaviour with amino acids, strain AMCB3 showed the greatest requirement for them and grew very well in their presence.

Regarding the use of amines, only AMCB1 presented a capacity to use them significantly, although did not show high grow with ethylamine.

Phenolic compounds were not used by any of the two strains.

The AMCB1 and AMCB2 strains were characterised genetically.

The sequencing of RNAr 16S: AMCB1

After the results obtained with the sequencing of RNAr 16S, strain AMCB1 was identified as Bacillus megaterium.

### Sequencing of RNAr 16S: AMCB3

In view of the results obtained, strain AMCB3 was identified as Bacillus subtilis.

According to the above, the applicants of the present invention isolated two strains, AMCB1 and AMCB3, which belong to the species Bacillus megaterium and Bacillus subtillis respectively, and which are accelerators of the composting processes.

Likewise, the invention relates to the compositions which include various combinations of these strains and/or other strains which are compatible.

Specifically, it relates to a strain containing at least the bacterial strain of Bacillus megaterium AMCB1 along with the bacterial strain Bacillus subtillis AMCB3, which are composting processes accelerators.

It also relates to a composition containing at least the bacterial strain of Bacillus megaterium AMCB1 and the bacterial strain of Bacillus subtillis AMCB3, which are composting processes accelerators, and/or the strain Azospirillum brasilense; and/or Azotobacter chroococcum and/or Saccharomyces cerevisae, together with a nutritional reserve.

The compositions may be applied in solid or liquid form including adjuvants such as emulsifying agents and resuspension agents.

Solid compositions can be in the form of powder, granules or wettable powder, while liquid compositions can be in the form of aqueous or nonaqueous solutions, suspensions, dispersions or concentrated formulations.
The preferred concentration of micro-organisms in the solid formulation must be from 10³ to 10⁹ colony forming units per gram (CFU/g), preferably from 10⁵ to 10⁷ CFU/g. The preferred concentration of micro-organisms in the liquid formulation must be from 10³ to 10⁹ colony forming units per millilitre (CFU/ml), preferably from 10⁵ to 10⁷ CFU/ml. However, these ranges may vary according to the residue to be treated, among other factors.

In these formulations the "carrier" or vehicle constitutes a nutritional reserve for the micro-organisms, as well as providing them with the protection required for a greater survival to colonise the residue in question.

Success in production of an important biomass of the various micro-organisms depends on the development of a suitable medium, which may be a cheap agricultural by-product, easily obtainable and with the suitable nutrient balance.

The use of vegetable biomass in fermentation processes as a source of renewable energy and raw material for industry is one of the fields of greatest interest in biotechnology. Within vegetable biomass, agricultural waste constitutes the most suitable lignocellulosic material due to their low cost and high availability. These substrates include molasses, seed flour (cotton, soy, etc.), hay, wheat, corn, barley, etc.

At the end of a liquid or solid fermentative process, biomass is collected at the same time as the substrate, the latter providing physical protection and a nutritional base for the micro-organism.

The composition of the invention can also be used as a fertilizer due to the good PGPR activity shown by strains AMCB1 and AMCB3.

### DESCRIPTION OF THE DRAWINGS

The present descriptive memory is completed by a set of drawings that illustrate the preferred example and in no way limit the invention.
Figure 1 shows the paired total ion chromatograms (TIC) of the pyrolysis products obtained by thermal desorption and flash pyrolysis at 300°C resulting from the analysis of compost samples of control stack (A) and the treated stack (B) in the first example of embodiment. Abundance is plotted vs. time in minutes.
Figure 2 shows the paired total ion chromatograms (TIC) of the products obtained by pyrolysis at 500°C resulting from the analysis of compost samples of control stack (A) and the treated stack (B) in the first example of embodiment. Abundance is plotted vs. time in minutes.
Figure 3 shows the paired total ion chromatograms (TIC) of the products obtained by direct pyrolysis at 500°C resulting from the analysis of compost samples of control stack (A) and the treated stack (B) in the first example of embodiment. Abundance is plotted vs. time in minutes.
Figure 4 shows the paired total ion chromatograms (TIC) of the pyrolysis products obtained by thermal desorption and flash pyrolysis at 300°C resulting from the analysis of compost samples of the control ridge (C) and the treated ridge (D) in the second example of embodiment. Abundance is plotted vs. time in minutes.
Figure 5 shows the total ion chromatograms of the products obtained by flash pyrolysis at 500°C for the second example of embodiment for both the control ridge (C) and the treated ridge (D). Abundance is plotted vs. time in minutes.
Figure 6 shows a reproducibility test for the results of the second example of embodiment. Abundance is plotted vs. time in minutes.
Figure 7 shows the total ion chromatograms (TIC) of the pyrolysis products obtained by thermal desorption and flash pyrolysis at 300°C resulting from the analysis of compost samples of the control ridge (E) and the treated ridges (F, G) in the third example of embodiment. Abundance is plotted vs. time in minutes.
Figure 8 shows the total ion chromatograms (TIC) of the pyrolysis products obtained by thermal desorption and flash pyrolysis at 500°C resulting from the analysis of compost samples of the control ridge (E) and the treated ridges (F, G) for the third example of embodiment. Abundance is plotted vs. time in minutes.
Figure 9 shows a comparison of the pyrograms for the control compost (top) and the treated compost (I) collected in May (bottom). Abundance is plotted vs. time in minutes.
Figure 10 shows a comparison of the pyrograms for the control compost (top) and the treated compost (I) collected in June (bottom). Abundance is plotted vs. time in minutes.
Figure 11 shows a comparison of the pyrograms for the control compost (top) and the treated compost (J) collected in May (bottom). Abundance is plotted vs. time in minutes.
Figure 12 shows a comparison of the pyrograms for the control compost (top) and the treated compost (J) collected in June (bottom). Abundance is plotted vs. time in minutes.

### PREFERRED EMBODIMENT OF THE INVENTION

In view of the above, the present invention consists of a composition for acelerating composting processes.

In one example of embodiment, the present invention provides a liquid formulation with the following quantitative composition:

| | | |
|---|---|---|
| □ | Molasses | 40-100 g/l |
| □ | Mn AEDT | 40-100 g/l |
| □ | Zn AEDT | 0.1-40 g/l |
| □ | Fe AEDT | 50-200 g/l |
| □ | Sodium molybdate | 0.1-1 g/l |
| □ | Boric acid | 0.1-1 g/l |
| □ | pH | 6.5-7.5 |
| □ | strains of the species Bacillus megaterium AMCB1 (CECT 7012); Bacillus subtilis AMCB3 (CECT 7014); Azospirillum brasilense; Azotobacter chroococcum and/or Saccharomyces cerevisae. | |

In another example of embodiment, the present invention provides a solid formulation composed by a solid support and a combination of biological components, with the following quantitative composition:

| | | |
|---|---|---|
| □ | Raw protein | 10-50 % |
| □ | Raw fatty matter | 10-30 % |
| □ | Cellulose | 1-10% |
| □ | Lactose | 10-60 % |
| □ | Copper sulphate (CuSO4.5H2O) | 5-20 mg/Kg |
| □ | pH | 6.5-7.5 |
| □ | strains belonging other species Bacillus megaterium AMCB1 (CECT 7012); Bacillus subtilis AMCB3 (CECT 7014); Azospirillum brasilense; Azotobacter chroococcum and/or Saccharomyces cerevisae. | |

### Example 1

A first example of the invention studies the application of the liquid composition during the composting process of waste water purification station sludge, to test its effectiveness in accelerating the process and reducing process operation tasks (turns).

The test was performed in the biosolids composting plant of the company EMASESA located in the southern waste-water purification station (Copero, province of Seville) on 24 September 2004.

The raw material used in this experience is sludge obtained from the municipal waste water purification stations of the company EMASESA.

After they are received the biosolids are dumped in the drying checks, extended by loading scoops and exposed to natural drying until their humidity level is 40-50% (the optimum humidity for the performance of micro-organisms).

After the optimum humidity is reached the extended matter is divided into two parts (≈11000 litres each) to form the control stack (A) and the treated stack (B).

The treatment with the liquid composition was performed in this stage, as the material is well extended so that an even application of the product can be ensured. An orientative initial dose of 3 litres of liquid composition was used initially (equivalent to 1.5 kg of the solid formula) per 11m³; these doses may be optimised afterward. A stock of the product was prepared with a minimum amount of water (7 litres) in order not to change the humidity of the raw material to be composted and it was sprayed on the extended sludge using a backpack sprayer with a 10 litre capacity. It was then piled up and turned to form semicircular stacks. The control stack (A) was prepared in the same conditions but was not treated with the composition.

Fermentation usually begins in this stage, and it must be maintained in suitable conditions by turns at variable frequency. A greater frequency of turns will accelerate the transformation by providing a greater aeration to the stacks. The aeration accelerates the decomposition of the mixtures, which generates high temperatures of 50-70°C, thereby allowing the pathogens present to be eliminated.

The composting process is completed with a maturation period which in normal conditions (without being treated with the composition) is extended for at least three months. In this last stage a greater decomposition, stabilisation, destruction of pathogens and degasification occur, thereby contributing to a final compost of higher quality.

Afterwards an optional drying and screening stage can be applied, ranging from a few days to several months.

To control the temperature in the stacks measurements were made in several areas of the stacks with a dial thermometer manufactured by MARTIN MARTENS.

The temperature readings of the control stack (A) and the treated stack (B) is provided in the following table. The composition was applied on 24/09/2004 and the turns were performed on 30/9/04, 14/10/04 and 29/10/04.

| Temperature reading date | Control stack (A) temperature in °C | Stack (B) temperature in °C |
|---|---|---|
| 24/09/04 | 31 | 32 |
| 27/09/04 | 31 | 33 |
| 28/09/04 | 33 | 32 |
| 29/09/04 | 33 | 32 |
| 30/09/04 | 33 | 33 |
| 1/10/04 | 36 | 37 |
| 04/10/04 | 37 | 37 |
| 05/10/04 | 38 | 38 |
| 06/10/04 | 37 | 39 |
| 07/10/04 | 37 | 40 |
| 08/10/04 | 37 | 42 |
| 11/10/04 | 38 | 43 |
| 13/10/04 | 38 | 44 |
| 14/10/04 | 29 | 31 |
| 15/10/04 | 34 | 45 |
| 18/10/04 | 35 | 46 |
| 19/10/04 | 35 | 47 |
| 20/10/04 | 36 | 44 |
| 21/10/04 | 36 | 45 |
| 22/10/04 | 37 | 46 |
| 25/10/04 | 38 | 46 |
| 24/09/04 | 31 | 32 |
| 27/09/04 | 31 | 33 |
| 28/09/04 | 33 | 32 |
| 29/09/04 | 33 | 32 |
| 30/09/04 | 33 | 33 |
| 1/10/04 | 36 | 37 |
| 04/10/04 | 37 | 37 |
| 05/10/04 | 38 | 38 |
| 06/10/04 | 37 | 39 |
| 07/10/04 | 37 | 40 |
| 08/10/04 | 37 | 42 |
| 11/10/04 | 38 | 43 |
| 13/10/04 | 38 | 44 |
| 26/10/04 | 37 | 45 |
| 27/10/04 | 36 | 44 |
| 28/10/04 | 34 | 46 |
| 29/10/04 | 27 | 32 |
| 02/11/04 | 34 | 44 |
| 03/11/04 | 34 | 43 |
| 04/11/04 | 34 | 42 |
| 05/11/04 | 33 | 42 |
| 08/11/04 | 33 | 41 |
| 09/11/04 | 33 | 41 |
| 10/11/04 | 33 | 41 |
| 11/11/04 | 34 | 41 |
| 12/11/04 | 33 | 41 |

The temperature readings were characterised by a favourable evolution in the treated stack (B) with the formulations. This increase was quick in comparison to the control stack (A). In fact, the temperature differences reached up to 12°C (19 October). The maximum temperature in the control stack (A) was 38°C, while in the stack (B) treated with the formulations it reached 47°C. After the first turn of the stacks the temperature increase was greater in the treated stack (B) than in the control stack (A). The stack (B) treated with the formulations reached favourable temperatures for operation of thermophile micro-organisms (>45°C), allowing the pathogens present to be eliminated.

During the turns the differences were observed visually. In fact, in the treated stack (B) a strong fume emission was observed, due to the high temperatures reached (which was not seen in the control stack (A)), as well as a strong fermentation odour. Another important fact is that the experiment took place during days with heavy rainfall, which did not prevent the favourable development of the process in the stack treated with the formulations.

The method employed was pyrolysis with gas chromatography - mass spectrometry (Py-GC-MS), for a direct characterisation of the organic matter (OM) present in complex matrices.

In the specific case of compost composition studies, it was shown that analytic pyrolysis allows both the origin of the organic substrate and its degree of maturity to be differentiated.

In view of this, a direct study by Py-GC-MS was conducted of control compost samples and the same compost subjected to treatment with the formulation and at a different degree of maturity, to detect possible changes in the characteristics of its OM (organic matter).

Likewise, another method was used: "desorption", fundamentally similar to pyrolysis. While pyrolysis is performed at a temperature of around 500°C, desorption takes place at a lower temperature (300°), allowing to obtain molecular information on the decomposition of very sensitive OM precursors.
Analyses using pyrolysis (Py) were performed with a PY2020 (Frontier Lab) pyrolyser associated to an Agilent 5973 gas chromatography - mass spectrometry (GC-MS) unit. The pyrolysis system (double-shot) allows a sequential pyrolysis at different temperatures on the same sample.

After performing several tests, optimised measurement conditions (for both chromatography and pyrolysis) were obtained for analysing compost samples, basically involving a first heating from 100 to 300°C (sub-pyrolysis temperature) at a rate of 30°C per minute, followed by a flash pyrolysis at 500°C for one minute. The first step can be considered a thermal desorption treatment, not a true pyrolysis, and it is suggested that it is especially suited for release by thermodesorption of volatile and semi-volatile compounds weakly associated to the substrate being analysed. The second phase (pyrolysis at 500°C) gives rise to the release of pyrolysis products obtained from the cracking of the organic macromolecules present in the matrix being analysed.

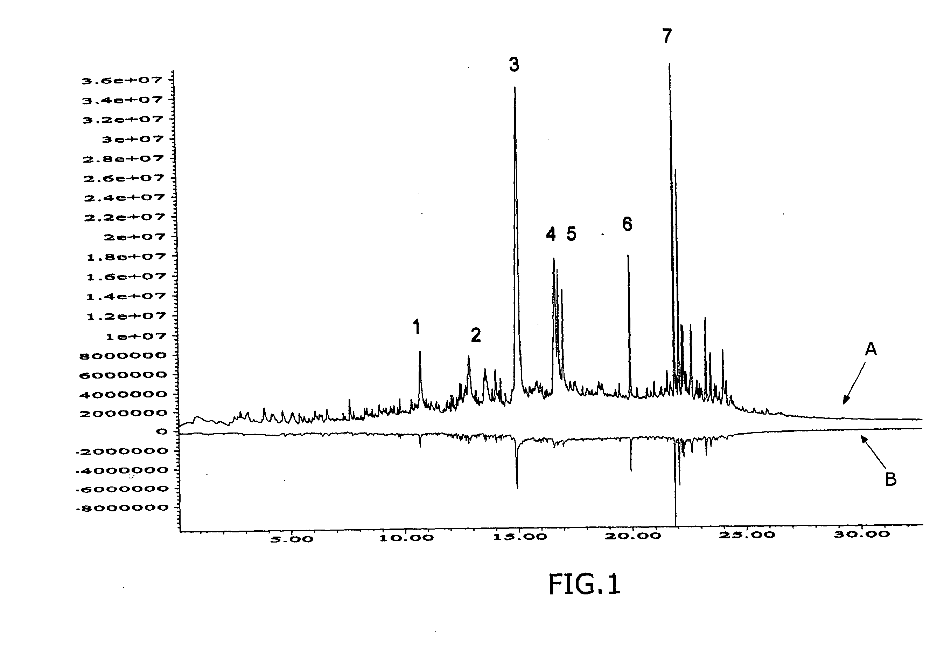

Pairs of compost samples were analysed from control sample (A) and treated sample (B) taken at one-week intervals in the months of September and October. The main alterations of the samples were found in the first week, and remained stable in the following samplings. A significant representation of the total ion chromatograms (TIC) of the pyrolysis products obtained by thermal desorption and flash pyrolysis (pyrograms), as well as by direct pyrolysis at 500°C obtained in the analysis of the control and treated compost samples is provided in figures 1-3.

Figure 1 shows the Py/desorption (300°C)-GC-MS analysis chromatograms for the control pairs (A) and treated pairs (B) samples. Clear alterations, mainly of a quantitative type, can be seen in the pyrolytic profile of the samples. Specifically, the treatment results in a significant reduction in the abundance of long chain fatty acids (C¹⁴-C¹⁸). In addition, the abundant thermo evaporation of sterols of microbial origin is observed in both samples. Nearly all pyrolysis products were unequivocally identified by their mass spectrograms. Figure 1 shows the chemical identity of some significant pyrolysis products, such as dodecanoic acid (1), tetradecanoic acid (2), palmitic acid (3), oleic acid (4), stearic acid (5), dialkyl phthalate (6), 5-alpha cholestane (7).

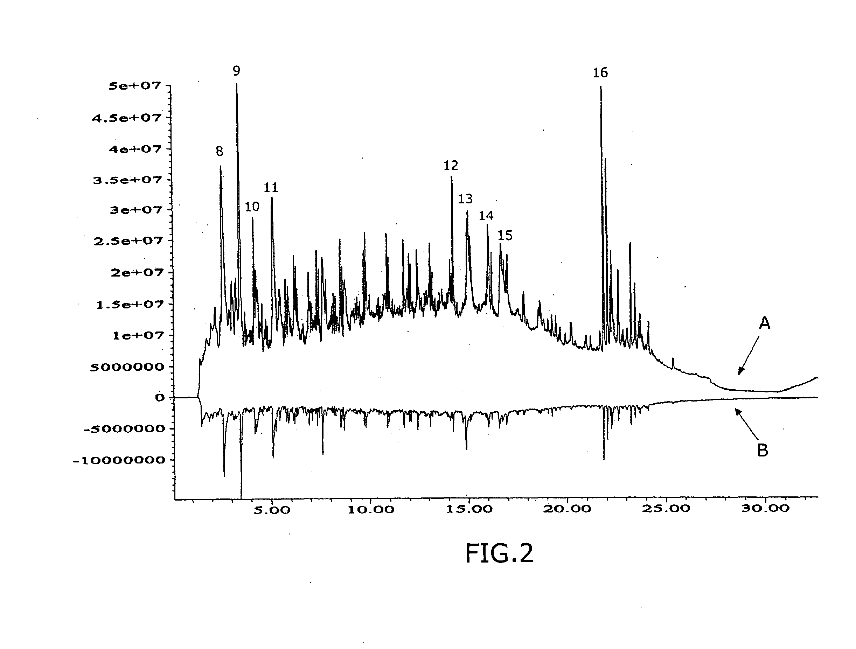

Figure 2 shows the Pyrolysis (500°C)-GC-MS analysis chromatograms where a similar behaviour is observed. This is, the quantitative differences between the pyrolysis products released in the control (A) and treated (B) samples, although the changes are less marked. The destruction of pyrolysis products indicates the presence of aliphatic biopolymers (alkane-alkene pairs), lipids (fatty acids) cellular walls (sterols), aromatic non-lignic components (toluene and phenols) among the components of both samples. In the treated sample the alkane-alkene pair pattern typical of the pyrolysis of aliphatic polymers is less clear, and the reduction of fatty acids mentioned above is equally significant. Figure 2 points out the chemical nature of representative pyrolysis products such as toluene (8), styrene (9), methylstyrene (10), methyl phenol (11), hexadecanitril (12), palmitic acid (13), oleic acid (14), steric acid (15) and 5-alpha cholestane (16).

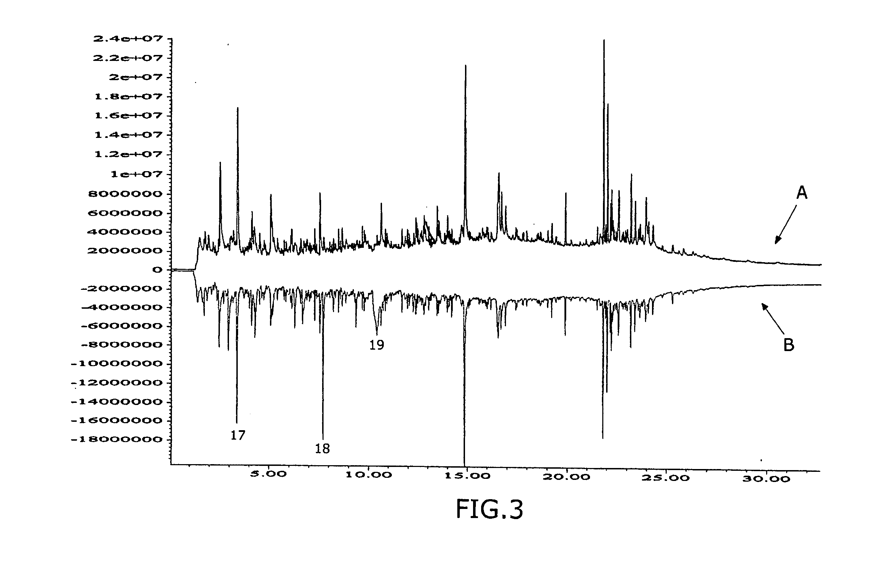

Figure 3 shows the paired direct pyrolysis (500°C)-GC-MS chromatograms, i.e. eliminating the prior phase of desorption at 300°C, confirming the trend observed in the sequential pyrolyses, particularly the reduction of fatty acids in the treated samples. A point worth noting is the apparent incorporation of pyrolysis products typical of proteins and polysaccharides in the treated sample (B), as shown by the appearance of furfural (17) and levoglucosane (19) and by the increased indene peak (18).

The results obtained by pyrolysis or desorption show an excellent reproducibility of pyrograms for samples taken one week after applying the formulations: control and treated pyrolysed on different days.

The results obtained allow the conclusion to be drawn that the use of biological formulations based on micro-organisms allows the acceleration of the composting process of waste water treatment plant sludge by approximately 50%.

The results of the pyrolysis analysis (Py-GC-MS) of the samples show that the decomposition of the various organic products obtained from polymers (lipids, polysaccharides, proteins, etc.) present in the sludge is much more effective when the formulations are applied compared to the untreated sample.

The acceleration of the waste water treatment plant sludge composting process by the performance of the formulation, has as a consequence, in addition to a better use of the space dedicated to the composting, reduced operation tasks (turns) and, therefore, lower costs.

### Example 2

In a second example of embodiment the application of the composition of the invention is studied during the composting of waste water treatment plant sludge mixed with vegetable biomass (VB), in order to test its effectiveness in accelerating the process and reducing operation tasks (turns).

The raw material used in this example is treatment plant sludge and vegetable biomass as a structure agent, obtained from forest and garden pruning. This last agent, aside from acting as a source of carbon during the compost formation, plays an essential role by increasing the porosity and therefore the contact area of the sludge exposed to oxygen during the process. It also reduces the initial humidity level of the mixture. In addition, the break-up of the matter increases its surface area exposed to microbial attack, affecting the speed of the process. A ratio of 1:3, i.e. one part sludge to three part structural agents, is considered adequate.

The sludge and biomass require a prior treatment. In the case of sludge, depending on their humidity it may or may not require a drying treatment performed in a number of checks provided for such a purpose.

The biomass must be in chips, obtained by grinding until fragments smaller than 15 cm long and 2 cm wide are obtained.

The ridge method is used, providing the following advantages: fast drying of the compost (humidity lost when the stacks are turned); drier compost material; possibility of handling a large volume of material; great stability of the product; low economic cost.

The various materials are deposited in layers for stacking or forming the ridge. Layers of the different raw materials are placed consecutively and alternately. After stacking, a first mixing is performed and fermentation begins.

The treatment with the formulations is performed during the first mixing of the stacked materials. A dose of 1 to 1.5 litres of bacterial formulation per 10 m³ of substrate is used initially. A broth of the product is prepared using a minimum amount of water and it is sprayed on the ridge, which is later turned to homogenise the treatment. Another ridge is prepared in the same conditions without applying the treatment with the formulation, in order to use it as a control.

At this stage the fermentation itself begins and is maintained in suitable conditions by turns applied with a variable frequency.

A greater turning frequency accelerates the transformation by providing a greater aeration of the ridges.

The composting process is completed with a maturing period which in normal conditions (without treatment with the formulation) will last at least two months. In this last phase, a greater decomposition, stabilisation, destruction of pathogens and degasification takes place, contributing to produce a final compost of greater quality.

The temperature readings of the control and treated ridges are characterised by a favourable evolution. However, this increase was very fast in the ridge treated with the formulation (the maximum temperatures obtained in the control ridge were between 50 and 52°C, while in the treated ridge they were 10 degrees higher). This is due to a correct aerobic fermentation process carried out by the inoculated micro-organisms.

In this example, in addition to a daily temperature reading, the composting process was monitored by the Py-GC-MS technique.

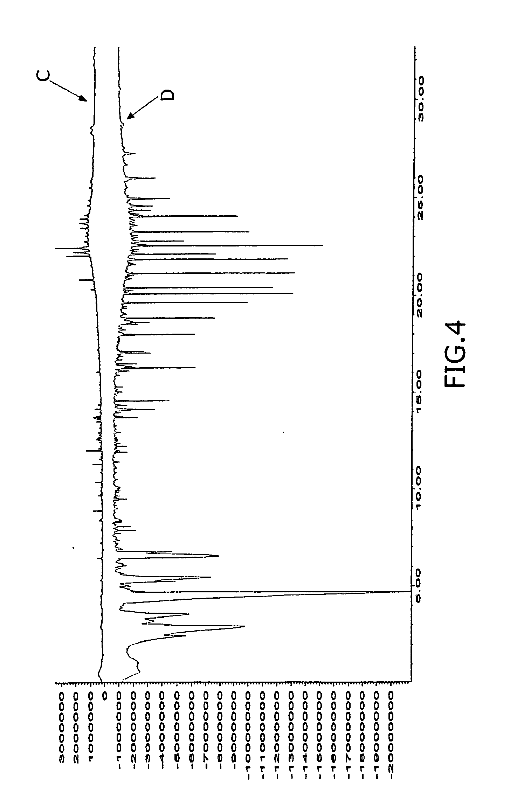

The total ion chromatograms (TIC) of the pyrolysis products obtained by thermal desorption and flash pyrolysis (pyrograms) in the analysis of the compost samples are shown in figures 4-5, and allow the following conclusions to be deduced:

Figure 4 shows a (300°C)-GC-MS Py/desorption analysis showing alterations in the pyrolytic profile of the control sample (C) and the treated sample (D), both in a qualitative and a quantitative sense. Treatment with the formulations causes a brusque change in the behaviour of the sample in thermal desorption, shown in the abundant thermo evaporation of aliphatic compounds (fatty acids with a low molecular mass and saturated straight-chain n-alkanes).

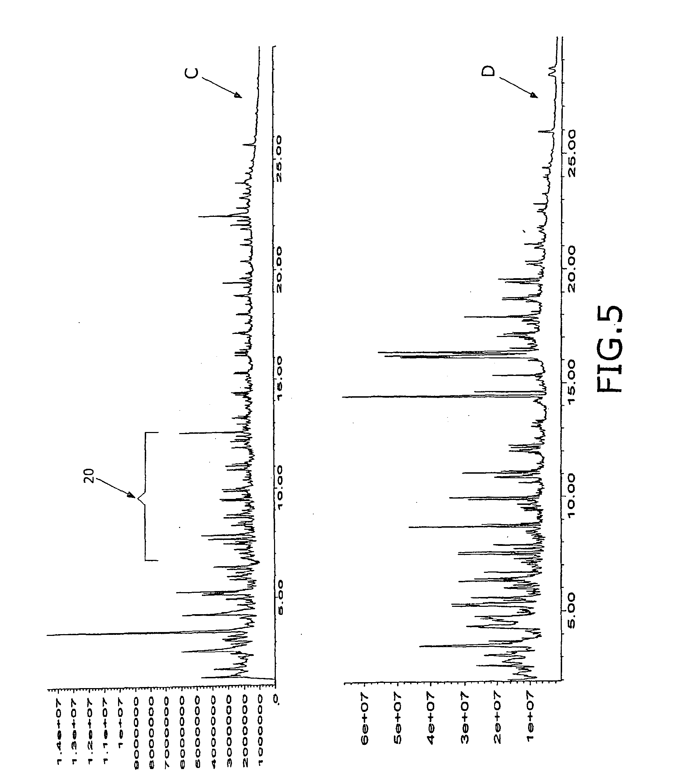

Figure 5 shows a pyrolysis (500°C)-GC-MS y analysis in which the aforementioned behaviour is reproduced, i.e. there are differences between the control sample (C) and the treated sample (D), although the changes are less marked. Molecular data indicate that the pyrogram of the control sample (C) clearly shows pyrolysis products of lignin (20). In the treated sample the lignin pattern is less obvious, and the pyrogram is dominated by aliphatic compounds with a high molecular mass (ketones, unsaturated fatty acids, etc.), probably from the microbial cell walls.

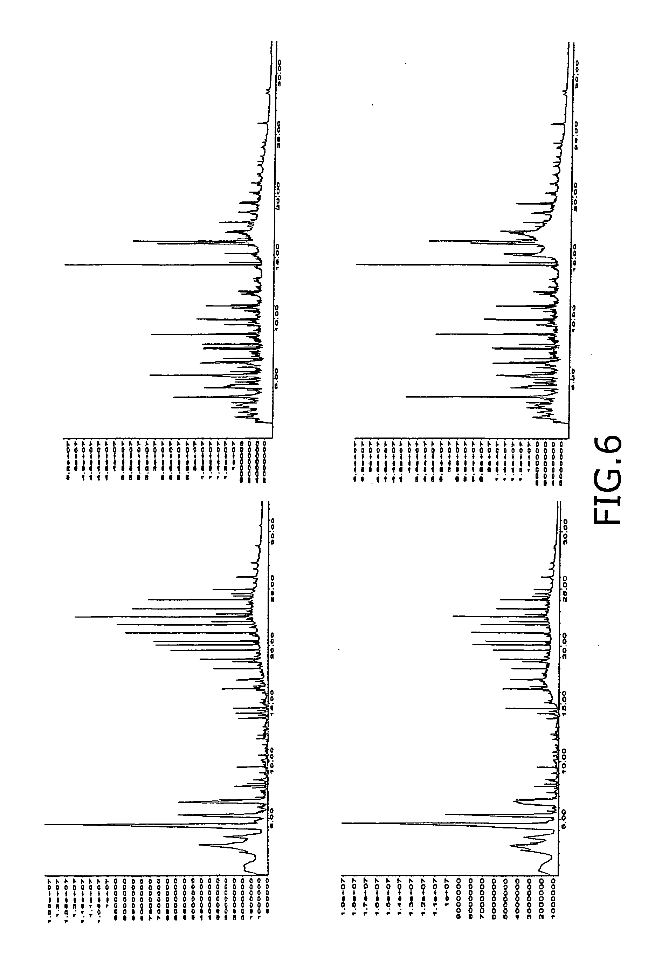

Figure 6 shows the excellent reproducibility of the pyrograms in the tests described above.

The results obtained as a whole allow the conclusion to be drawn that the use of biological formulations based on micro-organisms, object of the present invention, allows the composting process of waste obtained from waste water treatment plant sludge mixed with vegetable biomass to be accelerated (structural agent).

The results obtained from the Pyrolysis (Py-GC-MS) analysis show that the humidification of the various organic compounds obtained from polymers (lignins, polysaccharides, proteins, etc.), present in the sludge, is much more effective when the formulation is applied compared to the untreated control.

### Example 3

In a third example of embodiment, the raw material used is fresh municipal solid waste (MSW) supplied by the company EDIFESA- Seville. The organic fraction of the raw material (MSW) was selected, crushed and homogenised.

The different materials are deposited in layers for stacking or forming the ridge. After they are stacked a first mixing is performed and fermentation begins.

The treatment with the composition of this invention is performed during the previously described phase, specifically in the first mixing of the stacked materials. The initial dose is 1 litre of formulation per 10 m³. A stock of the product is prepared in 50 litres of water approximately and is sprayed on the ridge, which is then turned to homogenise the treatment. In our case, another ridge is prepared in the same conditions without treating with the formulation, in order to use it as a control.

Normally, at the end of the process an evaluation of the maturity and stability of the compost is performed that ensures its obtainment.

The monitoring of the temperature readings of the control and treated ridges is characterised by a favourable evolution. However, this increase has been very quick in the ridge treated with the composition that is the object of this patent (the maximum temperatures reached in the control ridge were from 60 to 62°C, while in the treated ridge they ranged from 65 to 70°C). This is due to a correct aerobic fermentative process performed by the micro-organisms inoculated in the waste.

The composting process in the control and treated ridges were monitored by the Py-GC-MS technique.

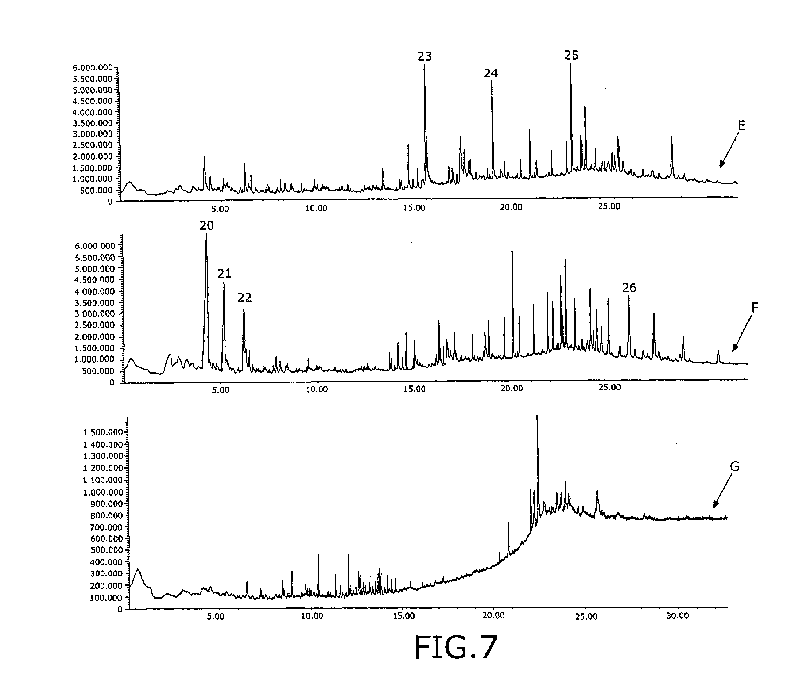

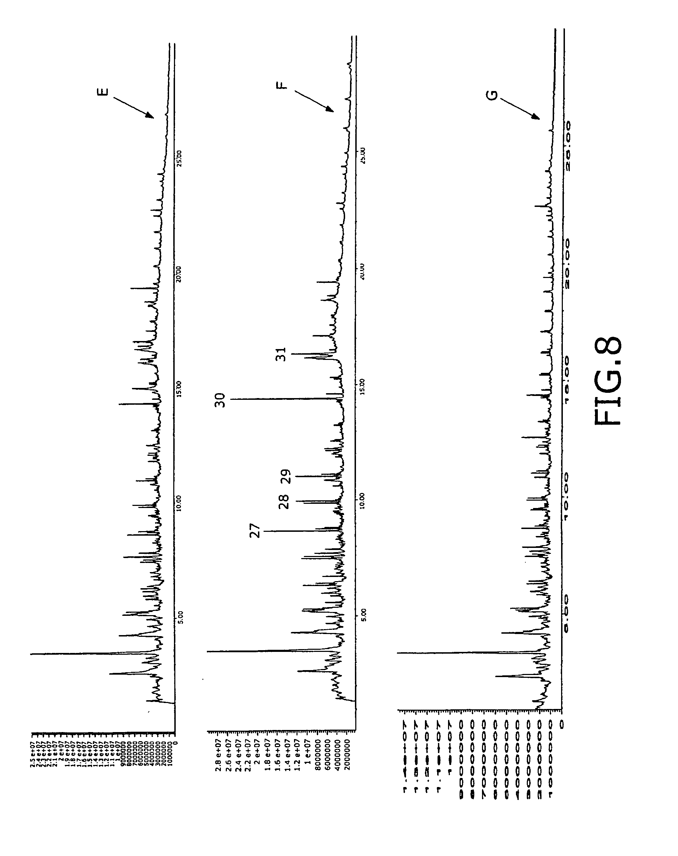

The results of the analysis of the samples of this compost are shown in figures 7 and 8. The following can be concluded in view of the pyrograms shown:

Figure 7 shows the thermal desorption chromatograms. Clear differences can be seen in the pyrolytic profiles of the control sample (E) and the treated samples (F, G). The pyrolytic behaviour of one of the treated samples (G) corresponds to a material of high stability, resulting from a great maturity.

The identity of the various peaks indicated in figure 7 is as follows: C₆-C₇ linear alkanoic acids (20-22); high molecular mass waxes (23,24); squalene (25); high molecular mass n-alkane (26).

Figure 8 shows chromatograms from pyrolysis at 500°C of the control sample (E) and the treated samples (F, G).

The chemical identity of the various peaks shown is the following: 1-3, C₁₄-C₁₆ alkane/alkene pairs (27-29), C₁₇-C₁₉ n-alkanones (30-31).

The results obtained from pyrolysis or desorption show an excellent reproducibility of pyrograms of samples taken 10 days after applying the formulation: control and treated pyrolysed on different days. Validation of the sample selection for analysis and the technique.

An evaluation of the results shown in the figures allows seeing that treatment with the formulations causes a brusque change in the pyrogram of the treated samples.

The interpretation of the molecular data allows important conclusions to be reached in relation to the degree of humidity of the matter during the process. Thus, in only 10 days great differences are observed between the control and the treated samples, with some peaks (phenols) appearing and others disappearing, indicating the gradual and quick decay of the lignocellulosic substrate, barely decayed in the control ridges.

### Example 4

In this example test stacks with a volume of 10 m³ were used containing vegetable waste matter from crops of Western Almeria, Spain. This volume allows simple handling of the stacks when turning and applying the various preparations to them.

The treatments applied are shown in the following table:

| Stack (10 m3) | Date of application | Dose of composition of the invention and Prog | Urea added (kg) |
|---|---|---|---|
| 1 | 15/05/03 | 100 cc | 7.5 |
| 2 | 16/05/03 | 1500 cc | |
| 3 | 16/05/03 | 1500 cc, Prog 1000cc | |
| 4 | 16/05/03 | 1500 cc and Prog 1000 cc | 7.5 |
| 5 | 16/05/03 | 1500 cc | 7.5 |
| 6 | 23/05/03 | 150 cc | 7.5 |

Prog refers to Progrowth, a product for potentiating microbial growth in soil manufactured by the applicant of this patent.

These treatments were made by dissolving the formulations in water to a final volume of 50 litres, the amount required for application to 1 m³ of vegetable material.

At different times during the composting process, samples of each treatment were taken, the temperature of the various stacks was measured daily and an analysis by Pyrolysis (Py-CG-MS) was subsequently performed of the samples taken throughout the process.

The following table gives the daily temperature readings in the various stacks.

| Date | Stack 1 | Stack 2 | Stack 3 | Stack 4 | Stack 5 | Stack 6 |
|---|---|---|---|---|---|---|
| 17/05/03 | 43 | 50.1 | 44.3 | 45.4 | 48.3 | |
| 19/05/03 | 45.3 | 51 | 46.3 | 47.8 | 49.2 | |
| 20/05/03 | 48.9 | 51.2 | 49.5 | 50.3 | 51.1 | |
| 21/05/03 | 50.5 | 51.8 | 53.3 | 52.7 | 53 | |
| 22/05/03 | 53.5 | 55.2 | 55.7 | 54.3 | 56.8 | 41.3 |
| 23/05/03 | 57.1 | 56.4 | 58.2 | 57.5 | 58.3 | 45.7 |
| 16/05/03 | 60 | 59.4 | 60.5 | 59 | 61.5 | 55.4 |
| 27/05/03 | 52.4 | 56.1 | 55.6 | 57.1 | 58.2 | 53.4 |
| 28/05/03 | 49.8 | 54.5 | 52.4 | 52.3 | 56.1 | 53.8 |
| 29/05/03 | 49.6 | 54.5 | 55.5 | 52.6 | 55.7 | 55.1 |
| 30/05/03 | 49.9 | 54.8 | 57.9 | 53.1 | 55.1 | 56.3 |
| 31/05/03 | 50.3 | 53 | 57.9 | 53.1 | 55.1 | 56.3 |
| 2/06/03 | 51.5 | 53.1 | 55.8 | 53.3 | 54.5 | 57 |
| 03/06/03 | 51.2 | 52.3 | 55.4 | 53.5 | 53.5 | 56.3 |
| 04/06/03 | 51.4 | 52.5 | 53.7 | 53 | 52.1 | 56.2 |
| 05/06/03 | 51.1 | 52 | 53.2 | 52.7 | 50.6 | 55.1 |
| 06/06/03 | 50.8 | 51.4 | 53.5 | 51.5 | 49.3 | 53.7 |
| 07/06/03 | 50.3 | 51.2 | 53 | 51.3 | 49.1 | 52.2 |
| 09/06/03 | 50.1 | 50.6 | 52.9 | 50.4 | 48.2 | 50.5 |
| 10/06/03 | 50 | 50.5 | 52.7 | 49.5 | 48.6 | 50.2 |
| Average | 50.84 | 53.08 | 53.55 | 52.49 | 53.29 | 52.7 |

A favourable evolution of the temperature was seen in all the stacks, verifying a fast increase of temperature with time as a result of a correct aerobic fermentation process performed by the various micro-organisms inoculated. Temperatures around 58-60 °C were reached in all cases, with a slightly higher reading (3-4°C) in stacks treated with the composition of the invention and Pro-growth. This temperature increase was very significant in stack 6, considering the difference in the time of inoculation (1 week later).

Subsequently (after 25/05/03), the stacks treated with the composition of the invention and Progrowth maintained a more constant, stable temperature, gradually falling until the process finished. In view of these results, it can be concluded that in the stacks treated with the composition of the invention and with Progrowth the fermentation remained active for a longer time than in the untreated stack. This positive effect brings about an improved quality of the compost (greater degradation of the polymers that constitute the initial vegetable material).

A direct study by Py-GC-MS was performed on control composts and the same compost subjected to various treatments in a different degree of maturity, in order to detect possible variations in the characteristics of its OM.

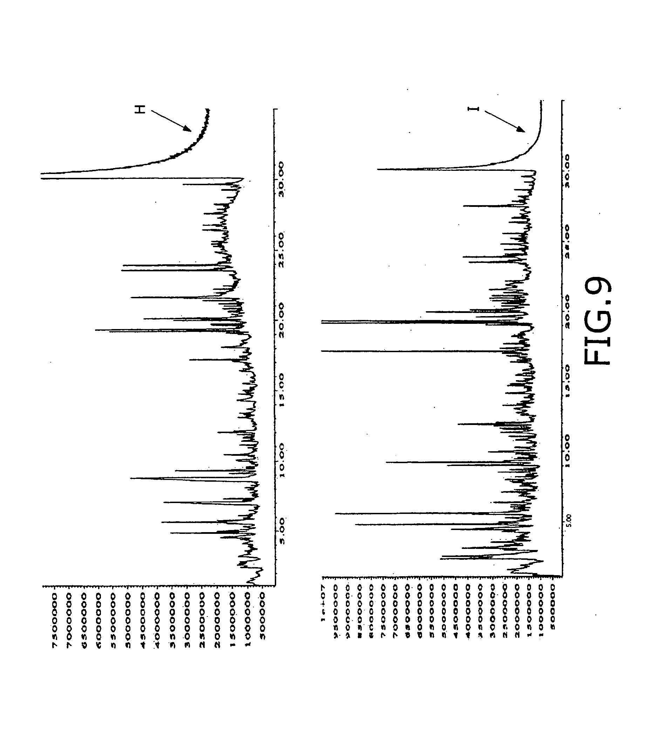

Figures 9-12 allow comparing the pyrograms of the control compost (which showed excellent reproducibility in various tests) and the treated composts collected at various maturing stages (I, J).

Despite their complexity, practically all the Pyrolysis products were identified, and with them their probable precursors. However, in this case it is more interesting to stress the obvious differences between the various patterns of products, particularly in the area of the pyrogram showing the most volatile products (approximately the first 15 minutes in the chromatography conditions used). Also obvious are the differences between the respective pyrograms of the treated composts (I, J) in different maturation stages.

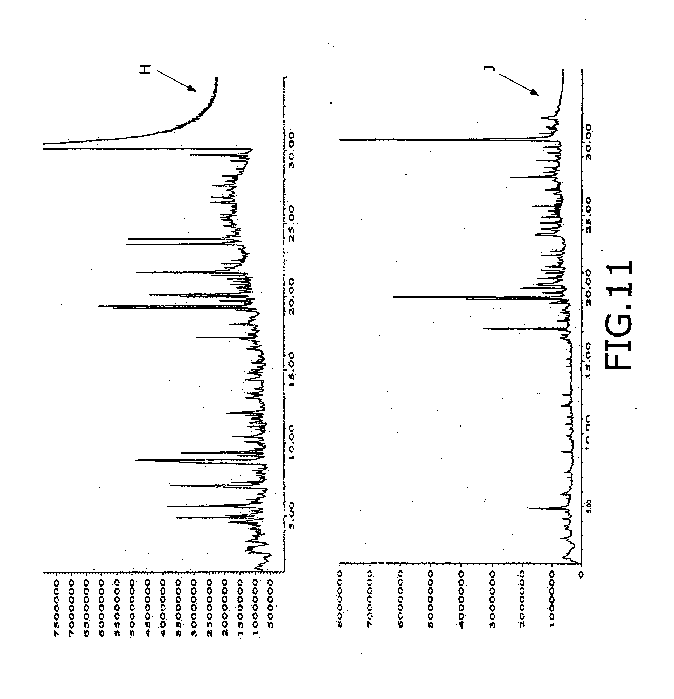

The bottom parts of figures 9 and 11 show the treated compost (I) collected in May and June respectively.

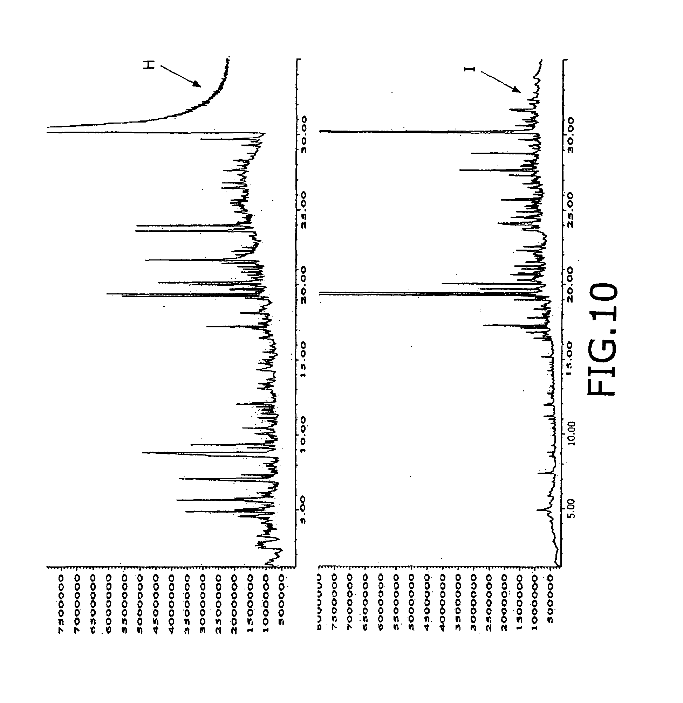

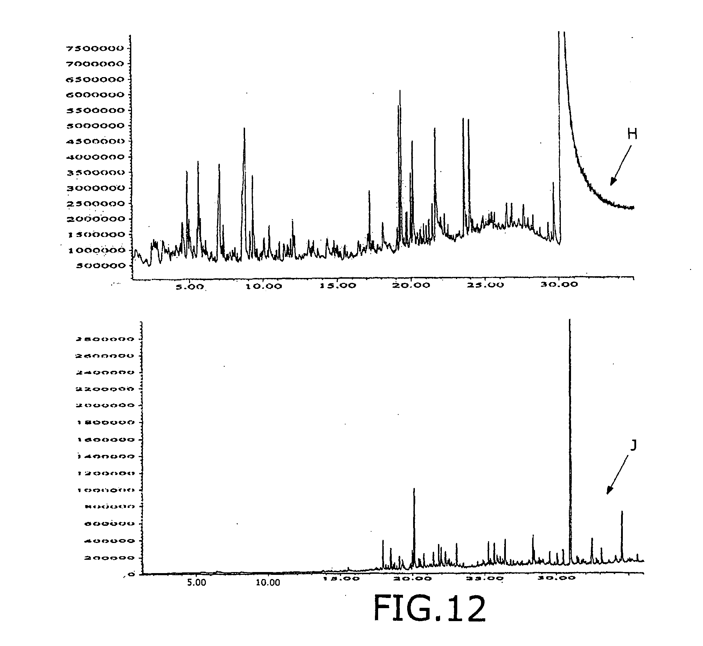

The bottom part of figures 10 and 12 show the treated compost (J) collected in May and June respectively.

The use of biological formulations based on micro-organisms object of this invention allows accelerating the composting process of the vegetable material. The comparative study has shown that the application of the formulations results in a suitable evolution of the temperatures that enable the fermentation processes responsible for a greater degradation of the vegetable material used to obtain the compost.

The results of the Pyrolysis analysis (Py-GC-MS) show that the humidification of the various organic compounds produced from polymers (such as lignins, polysaccharides, proteins, etc.) present in the vegetable matter was much more efficient when the formulations were applied. It is economically profitable to treat the compost with the bacterial formulation, as the increase in cost is not significant compared to the advantages obtained.

The essence of this invention is not affected by variations in the materials, shape, size and arrangement of the component elements, described in a non-limiting manner allowing its reproduction by an expert.

## Claims

1. Bacterial strain of Bacillus megaterium AMCB1 deposited in the Spanish Standard Culture Collection (Colección Española de Cultivos Tipo, CECT) on 27 July 2004 under the access number CECT 7012 which accelerates composting processes.

2. A composition **characterised in that** it comprises at least the bacterial strain of claim 1, together with the bacterial strain Bacillus subtillis deposited in the Spanish Standard Culture Collection (Colección Española de Cultivos Tipo, CECT) on 27 July 2004 under the access number CECT 7014, which accelerates composting processes.

3. A composition according to claim 2, **characterised in that** it contains strains of Azospirillum brasilense, Azotobacter chroococcum and Saccharomyces cerevisae, together with a nutritional reserve.

4. A composition according to claim 3, **characterised in that** the preferred concentration of the micro-organisms in a liquid formulation is 103 to 109 colony forming units per millilitre (cfu/ml).

5. A composition according to claim 4, **characterised in that** the preferred concentration of the micro-organisms in a liquid formulation is 105 to 107 colony forming units per millilitre (cfu/ml).

6. A composition according to claim 3, **characterised in that** the preferred concentration of the micro-organisms in a solid formulation is 103 to 109 cfu/g.

7. A composition according to claim 6, **characterised in that** the preferred concentration of the micro-organisms in a solid formulation is 105 to 107 cfu/g.

8. A composition according to claim 3, **characterised in that** the nutritional reserve at least contains:
| | |
|---|---|
| Molasses | 40-100 g/l |
| Mn AEDT | 40-100 g/l |
| Zn AEDT | 0.1-40 g/l |
| Fe AEDT | 50-200 g/l |
| Sodium molybdate | 0.1-1 g/l |
| Boric acid | 0.1-1 g/l |
| pH | 6.5-7.5 |

9. A composition according to claim 3, **characterised in that** the nutritional reserve at least contains:
| | |
|---|---|
| Raw protein | 10-50 % |
| Raw fatty matter | 10-30 % |
| Cellulose | 1-10 % |
| Lactose | 10-60 % |
| Copper sulphate (Cu SO4. 5H2O) | 5-20mg/Kg |
| pH | 6.5-7.5 |

10. Use of the composition according to claims 2 to 9 to accelerate composting.

11. Use according to claim 10, **characterised in that** it accelerates the composting process of waste water treatment plant sludge.

12. Use according to claim 10, **characterised in that** it accelerates the composting process of waste water treatment plant sludge mixed with vegetable biomass.

13. Use according to claim 10, **characterised in that** it accelerates the composting process of municipal solid waste (MSW).

14. Use according to claim 10, **characterised in that** it accelerates the composting process of vegetable matter.

15. Use of the composition according to claims 2 to 9 as a fertiliser.

## Patentansprüche

1. Bakterienstrang des am 27. Juli 2004 bei der spanischen Sammlung von Mikroorganismen und Zellkulturen (Colección Española de Cultivos Tipo, CECT) unter der Zugangsnummer CECT 7012 hinterlegten Bacillus megaterium AMCB1, welcher Kompostierprozesse beschleunigt.

2. Eine Zusammensetzung, **dadurch gekennzeichnet, dass** sie mindestens einen Bakterienstrang nach Anspruch 1 umfasst, zusammen mit dem Bakterienstrang des am 27. Juli 2004 bei der spanischen Sammlung von Mikroorganismen und Zellkulturen (Colección Española de Cultivos Tipo, CECT) unter der Zugangsnummer CECT 7014 hinterlegten Bacillus subtilis, welcher Kompostierprozesse beschleunigt.

3. Eine Zusammensetzung nach Anspruch 2, **dadurch gekennzeichnet, dass** sie Stränge von Azospirillum brasilense, Azotobacter chroococcum und Saccharomyces cerevisae, zusammen mit einer Nährreserve enthält.

4. Eine Zusammensetzung nach Anspruch 3, **dadurch gekennzeichnet, dass** die bevorzugte Konzentration der Mikroorganismen in einer flüssigen Formulierung 103 bis 109 Kolonien bildende Einheiten pro Milliliter (KbE/ml) beträgt.

5. Eine Zusammensetzung nach Anspruch 4, **dadurch gekennzeichnet, dass** die bevorzugte Konzentration der Mikroorganismen in einer flüssigen Formulierung 105 bis 107 Kolonien bildende Einheiten pro Milliliter (KbE/ml) beträgt.

6. Eine Zusammensetzung nach Anspruch 3, **dadurch gekennzeichnet, dass** die bevorzugte Konzentration der Mikroorganismen in einer festen Formulierung 103 bis 109 Kolonien bildende Einheiten pro Milliliter (KbE/ml) beträgt.

7. Eine Zusammensetzung nach Anspruch 6, **dadurch gekennzeichnet, dass** die bevorzugte Konzentration der Mikroorganismen in einer festen Formulierung 105 bis 107 Kolonien bildende Einheiten pro Milliliter (KbE/ml) beträgt.

8. Eine Zusammensetzung nach Anspruch 3, **dadurch gekennzeichnet, dass** die Nährreserve mindestens die folgenden Bestandteile enthält:
| | |
|---|---|
| Melasse | 40 - 100 g/l |
| Mn AEDT | 40 - 100 g/l |
| Zn AEDT | 0,1 - 40 g/l |
| Fe AEDT | 50 - 200 g/l |
| Natriummolybdate | 0,1 - 1 g/l |
| Borsäure | 0,1 - 1 g/l |
| pH | 6,5 - 7,5 |

9. Eine Zusammensetzung nach Anspruch 3, **dadurch gekennzeichnet, dass** die Nährreserve mindestens die folgenden Bestandteile enthält:
| | |
|---|---|
| Rohprotein | 10 - 50% |
| Rohfettgehalt | 10 - 30% |
| Zellulose | 1 - 10% |
| Laktose | 10 - 60% |
| Kupfersulfat (CuSO_{4.} * 5 H₂O) | 5 - 20 mg/kg |
| pH | 6,5 - 7,5 |

10. Verwendung der Zusammensetzung nach den Ansprüchen 2 bis 9 zur Beschleunigung der Kompostierung.

11. Verwendung nach Anspruch 10, **dadurch gekennzeichnet, dass** sie den Kompostierprozess von Wasseraufbereitungsschlamm beschleunigt.

12. Verwendung nach Anspruch 10, **dadurch gekennzeichnet, dass** sie den Kompostierprozess von Wasseraufbereitungsschlamm gemischt mit pflanzlicher Biomasse beschleunigt.

13. Verwendung nach Anspruch 10, **dadurch gekennzeichnet, dass** sie den Kompostierprozess von Hausmüll beschleunigt.

14. Verwendung nach Anspruch 10, **dadurch gekennzeichnet, dass** sie den Kompostierprozess von pflanzlichem Material beschleunigt.

15. Verwendung der Zusammensetzung nach den Ansprüchen 2 bis 9 als Düngemittel.

## Revendications

1. Souche bactérienne de Bacillus megaterium AMCB1 déposée à la Collection Espagnole de Cultures Type (Colección Española de Cultivos Tipo, CECT) le 27 juillet 2004 sous le numéro d'accès CECT 7012 qui accélère les processus de compostage.

2. Une composition **caractérisée en ce qu'**elle comprend au moins la souche bactérienne de la revendication 1, conjointement avec la souche bactérienne Bacillus subtillis déposée à la Collection Espagnole de Cultures Type (Colección Española de Cultivos Tipo, CECT) le 27 juillet 2004 sous le numéro d'accès CECT 7014 qui accélère les processus de compostage.

3. Une composition selon la revendication 2, **caractérisée en ce qu'**elle contient des souches d'Azospirillum brasilense, d'Azotobacter chroococcum et de Saccharomyces cerevisiae, conjointement avec une réserve nutritionnelle.

4. Une composition selon la revendication 3, **caractérisée en ce que** la concentration préférée des micro-organismes dans une formulation liquide est de 103 à 109 unités formant colonie par millilitre (ufc/ml).

5. Une composition selon la revendication 4, **caractérisée en ce que** la concentration préférée des micro-organismes dans une formulation liquide est de 105 à 107 unités formant colonie par millilitre (ufc/ml).

6. Une composition selon la revendication 3, **caractérisée en ce que** la concentration préférée des micro-organismes dans une formulation solide est de 103 à 109 ufc/g.

7. Une composition selon la revendication 6, **caractérisée en ce que** la concentration préférée des micro-organismes dans une formulation solide est de 105 à 107 ufc/g.

8. Une composition selon la revendication 3, **caractérisée en ce que** la réserve nutritionnelle contient au moins :
| | |
|---|---|
| Mélasse | 40-100 g/l |
| Mn AEDT | 40-100 g/l |
| Zn AEDT | 0,1-40 g/l |
| Fe AEDT | 50-200 g/l |
| Molybdate de sodium | 0,1-1 g/l |
| Acide borique | 0,1-1 g/l |
| pH | 6,5-7,5 |

9. Une composition selon la revendication 3, **caractérisée en ce que** la réserve nutritionnelle contient au moins :
| | |
|---|---|
| Protéine brute : | 10-50% |
| Matière grasse brute | 10-30% |
| Cellulose | 1-10% |
| Lactose | 10-60% |
| Sulfate de cuivre (Cu SO4. 5H2O) | 5-20 mg/Kg |
| pH | 6,5-7,5 |

10. Utilisation de la composition selon les revendications 2 à 9 pour accélérer le compostage.

11. Utilisation selon la revendication 10, **caractérisée en ce qu'**elle accélère le processus de compostage de la boue des installations de traitement des eaux résiduaires.

12. Utilisation selon la revendication 10, **caractérisée en ce qu'**elle accélère le processus de compostage de la boue des installations de traitement des eaux résiduaires mélangée avec la biomasse végétale.

13. Utilisation selon la revendication 10, **caractérisée en ce qu'**elle accélère le processus de compostage des résidus solides urbains (RSU).

14. Utilisation selon la revendication 10, **caractérisée en ce qu'**elle accélère le processus de compostage des matières végétales.

15. Utilisation de la composition selon les revendications 2 à 9 comme engrais.
